# EUROPÄISCHE PATENTSCHRIFT

(11) **EP 3 340 095 B1**
(45) Veröffentlichungstag und Bekanntmachung des Hinweises auf die Patenterteilung: **08.07.2020**
(21) Anmeldenummer: 17002040.8
(22) Anmeldetag: 20.12.2017
(51) Int. Cl.: G06F 21/31, H04L 29/06

(54) **BEATMUNGSSYSTEM UND VERFAHREN**
VENTILATION SYSTEM AND METHOD
SYSTÈME D'ASSISTANCE RESPIRATOIRE ET PROCÉDÉ

(30) Priorität: 23.12.2016 DE 102016015440
(43) Veröffentlichungstag der Anmeldung: 27.06.2018
(73) Patentinhaber: Löwenstein Medical Technology S.A., 2557 Luxembourg (LU)
(72) Erfinder: Schwaibold, Matthias, 76228 Karlsruhe (DE)
(74) Vertreter: Marx, Thomas

(56) Entgegenhaltungen:
- DE-A1-102009 008 070
- US-A1- 2014 123 237
- US-A1- 2015 237 222

## Beschreibung

Die vorliegende Erfindung betrifft ein Verfahren zum Betreiben wenigstens einer Datenverarbeitungseinrichtung eines Beatmungssystems sowie ein Beatmungssystem mit wenigstens einer Datenverarbeitungseinrichtung. Das Beatmungssystem umfasst wenigstens ein Beatmungsgerät und wenigstens eine räumlich von dem Beatmungsgerät getrennte Gegenstelle. Mit der Datenverarbeitungseinrichtung werden Therapiedaten zwischen dem Beatmungsgerät und der Gegenstelle übertragen.

Beatmungsgeräte bzw. Schlaftherapiegeräte verfügen in der Regel über eine Speichereinrichtung, um Therapiedaten hinterlegen und abrufen zu können. So werden z. B. die zur Therapie einer bestimmten Atemstörung notwendigen Beatmungsparameter hinterlegt. Oft werden auch Daten gespeichert, welche den Therapieverlauf charakterisieren.

Aus dem Dokument US 2015/237 222 A1 ist ein Verfahren zum Betreiben einer Bildgebungsmodalität für Magnetresonanzsysteme oder Röntgengeräte bekannt, wobei die Bildgebungsmodalität mindestens eine Steuereinheit umfasst.

Aus dem Dokument DE 10 2009 008 070 A1 ist ein medizintechnisches Gerät mit zumindest einem Sensor zur Erfassung von zumindest einem Parameter des Atemgases bekannt.

Aus dem Dokument US 2014/123 237 ist ein Verfahren zum Freigeben von Inhalten, auf den auf autorisierte Benutzer innerhalb einer über ein Netzwerk verwalteten Benutzergruppe zugegriffen wird.

Solche Daten sind besonders hilfreich, um einen Erfolg der Therapie beurteilen zu können oder um eine bestimmte Diagnose zu unterstützen. Zudem liefern die Daten auch Auskunft darüber, ob der Benutzer das Beatmungsgerät korrekt und regelmäßig anwendet. Um eine zuverlässige Diagnose und eine wirksame Therapie gewährleisten zu können, ist daher eine regelmäßige bzw. möglichst häufige Beurteilung der Therapiedaten besonders wichtig.

Es sind daher Beatmungsgeräte bekannt geworden, auf deren Speicher von außerhalb zugegriffen werden kann, sodass zur Begutachtung der Therapiedaten keine Besuche nötig sind. Besonders vorteilhaft ist das bei Beatmungssystemen mit mehreren Beatmungsgeräten bzw. Patienten.

Beispielsweise ist ein Zugriff auf die gespeicherten Daten möglich, sobald eine Gegenstelle mit einer geeigneten Software vorhanden ist, die die Daten interpretieren kann. Dies stellt jedoch eine erhebliche Schwäche in Bezug auf Datenschutz und Datensicherheit dar.

Bei einigen Beatmungsgeräten muss daher auch die Seriennummer des Geräts angegeben werden, um von der Gegenstelle aus zugreifen zu können. Dadurch soll gewährleistet werden, dass sich das Gerät mit der eingegebenen Seriennummer örtlich beim Benutzer befindet. Damit ist jedoch noch immer nicht ausreichend sichergestellt, dass kein Fehler bei der Eingabe vorliegt oder sich der Nutzer Zugriff zu einem Gerät verschaffen möchte, zu dem er keine Berechtigung besitzt.

Daher wird bei einigen Beatmungsgeräten manchmal eine zweite Nummer als Gerätecode auf das Gerät aufgedruckt. Ein Gerätezugriff kann nur eingerichtet werden, wenn Seriennummer und Gerätecode gemeinsam richtig eingegeben werden. Allerdings hat auch diese Lösung erhebliche Nachteile. Personen, die zu einem früheren Zeitpunkt das Gerät vor sich hatten, könnten den Gerätecode notiert oder abfotografiert haben. Personen, die zu einem späteren Zeitpunkt das Gerät vor sich haben, können sich ebenfalls noch unbemerkt Zugriff verschaffen, ob sie legitimiert sind oder nicht.

Es ist daher die Aufgabe der vorliegenden Erfindung, ein Verfahren zum Betreiben einer Datenaufbereitungseinrichtung eines Beatmungssystems und ein solches Beatmungssystem zur Verfügung zu stellen, mit denen ein verbesserter Zugriff auf Therapiedaten möglich ist. Insbesondere soll der Zugriff in Bezug auf Datenschutz und Datensicherheit verbessert werden.

Diese Aufgabe wird mit dem Verfahren des Anspruchs 1 sowie mit dem Beatmungssystem gemäß Anspruch 22 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Das erfindungsgemäße Verfahren dient zum Betreiben wenigstens einer Datenverarbeitungseinrichtung wenigstens eines Beatmungssystems. Das Beatmungssystem umfasst wenigstens ein Beatmungsgerät und wenigstens eine räumlich von dem Beatmungsgerät getrennte Gegenstelle. Mit der Datenverarbeitungseinrichtung sind Therapiedaten zwischen dem Beatmungsgerät und der Gegenstelle wenigstens teilweise übertragbar. Von wenigstens einem Benutzer des Beatmungssystems wird wenigstens ein Berechtigungscode in der Datenverarbeitungseinrichtung hinterlegt, um über die Gegenstelle einen berechtigten Zugriff auf die Therapiedaten zu erhalten. Dabei wird der Berechtigungscode dem Benutzer erst dann zur Verfügung gestellt, wenn dieser den Berechtigungscode durch wenigstens eine gegenüber der Datenverarbeitungseinrichtung und insbesondere gegenüber der Beatmungseinrichtung und/oder der Gegenstelle vorzunehmende Benutzeraktion anfordert.

Die Erstellung durch die Gegenstelle setzt insbesondere voraus, dass der Benutzer wenigstens einen legitimierten Benutzeraccount bei der Gegenstelle besitzt und insbesondere sich dort vorab authentifiziert. Der zur Verfügung gestellte Berechtigungscode identifiziert den Benutzer bevorzugt eindeutig.

Das erfindungsgemäße Verfahren bietet viele Vorteile. Ein erheblicher Vorteil ist, dass der Berechtigungscode dem Benutzer nur dann zur Verfügung gestellt wird, wenn dieser diesen anfordert und dazu wenigstens eine Benutzeraktion vornimmt. Dadurch ist ein in Bezug auf Datenschutz und Datensicherheit erheblich zuverlässiger Zugriff auf die Therapiedaten möglich. So ist der Berechtigungscode von außen am Gerät nicht sichtbar, bevor das Gerät in Betrieb genommen wird und die Ausgabe des Berechtigungscodes angefordert wird.

Insbesondere erhält der Benutzer anhand des Berechtigungscodes einen Zugriff auf das Beatmungsgerät und vorzugsweise auf die dort hinterlegten Therapiedaten. Insbesondere kann der Benutzer auf dem Beatmungsgerät abgelegte Therapiedaten von der Gegenstelle aus einsehen und/oder bearbeiten, zum Beispiel auswerten, verändern, kopieren und/oder verschieben.

Ohne Berechtigung ist insbesondere kein Zugriff auf die Therapiedaten möglich. Insbesondere ist ein Zugriff auf die Therapiedaten nur bei einer Verwendung des zur Verfügung gestellten Berechtigungscode möglich.

Hat der Benutzer einen berechtigten Zugriff, kann beispielsweise ein direkter Zugriff auf die Therapiedaten vorgesehen sein, die auf dem Beatmungsgerät gespeichert sind. Die Therapiedaten können aber auch wenigstens zeitweise in der Gegenstelle und z. B. auf einem Server hinterlegt sein, sodass der Zugriff auf die Therapiedaten des Beatmungsgerätes indirekt über die Gegenstelle erfolgt.

Das Hinterlegen des Berechtigungscodes in der Datenverarbeitungseinrichtung kann wenigstens teilweise manuell durch den Benutzer erfolgen, z. B. durch Eingabe an wenigstens einem User Interface bzw. Benutzerschnittstelle und/oder durch Verbindung und/oder Einstecken eines Datenträgers am Beatmungsgerät, der den Berechtigungscode enthält. Möglich ist auch, dass das Hinterlegen des Berechtigungscodes wenigstens teilweise automatisch erfolgt, beispielsweise durch wenigstens eine Netzwerkübermittlung des Berechtigungscodes an das Beatmungsgerät und/oder die Gegenstelle und/oder durch wenigstens eine Erzeugung durch wenigstens eine Algorithmik im Beatmungsgerät. Insbesondere ist die Datenverarbeitungseinrichtung mit dem Beatmungsgerät und der Gegenstelle wirkverbunden, sodass das Hinterlegen des Berechtigungscodes in der Datenverarbeitungseinrichtung durch Hinterlegen des Berechtigungscodes im Beatmungsgerät und/oder der Gegenstelle erfolgen kann.

Der Berechtigungscode ist insbesondere ohne die durch die Benutzeraktion vorgenommene Anforderung für den Benutzer nicht einsehbar. Insbesondere ist der Berechtigungscode von außerhalb des Beatmungsgeräts nicht sichtbar und insbesondere nicht auf dem Beatmungsgerät aufgedruckt.

Insbesondere ist die Datenverarbeitungseinrichtung mit dem Beatmungsgerät und der Gegenstelle wirkverbunden, sodass eine gegenüber dem Beatmungsgerät oder der Gegenstelle vorgenommene Benutzerkation auch gegenüber der Datenverarbeitungseinrichtung vorgenommenen wird.

Besonders bevorzugt wird die Benutzeraktion wenigstens teilweise mit dem Beatmungsgerät durchgeführt. Insbesondere berechtigt der Berechtigungscode nur zu einem Zugriff auf Therapiedaten desjenigen Beatmungsgerätes, mit dem die Benutzeraktion durchgeführt wird. Dadurch kann zum Beispiel gewährleistet werden, dass der Benutzer auch nur auf die Beatmungsgeräte des Beatmungssystems zugreifen kann, mit denen er auch tatsächlich Umgang hat. Insbesondere wird der Berechtigungscode dazu wenigstens teilweise dem Beatmungsgerät zugeordnet. Die Zuordnung erfolgt beispielsweise über eine Seriennummer und/oder eine Geräteidentifikationsnummer oder dergleichen. Zusätzlich kann der Berechtigungscode dem Benutzer zugeordnet sein.

Insbesondere berechtigt der Berechtigungscode nur für einen Zugriff auf ein bestimmtes Beatmungsgerät und/oder auf nur ein einzelnes Beatmungsgerät. Möglich ist aber auch, dass der Berechtigungscode auch zu einem Zugriff auf weitere Beatmungsgeräte des Beatmungssystems berechtigt. Insbesondere sind dazu bestimmte Beatmungsgeräte einer Gruppe zugeordnet, wobei der Berechtigungscode für einen Zugriff auf den Beatmungsgeräten der Gruppe berechtigt. Das ist beispielsweise für Betreiber von größeren Beatmungssystemen von großem Vorteil.

Die Benutzeraktion umfasst insbesondere wenigstens eine Inbetriebnahme des Beatmungsgeräts. Vorzugsweise umfasst die Benutzeraktion wenigstens eine Eingabe in das Beatmungsgerät. Für die Eingabe weist das Beatmungsgerät vorzugsweise wenigstens eine Benutzerschnittstelle auf. Die Eingabe kann beispielsweise die Auswahl einer Gerätefunktion umfassen, welche die Ausgabe oder Generierung des Berechtigungscodes startet. Zum Beispiel kann eine Menüeingabe und/oder eine Texteingabe vorgesehen sein. Möglich ist auch, dass die Benutzeraktion wenigstens teilweise gegenüber der Gegenstelle und insbesondere wenigstens eine Eingabe in eine Benutzerschnittstelle der Gegenstelle umfasst.

Die Benutzeraktion kann wenigstens eine biometrische Erfassung des Benutzers und/oder wenigstens ein Sprachkommando und/oder wenigstens eine Kopplung des Beatmungsgeräts mit wenigstens einem Speichermedium umfassen. Die biometrische Erfassung kann beispielsweise wenigstens eine Erfassung wenigstens eines Fingerabdrucks und/oder wenigstens einer Irisstruktur umfassen. Insbesondere wird wenigstens ein transportables bzw. entnehmbares Speichermedium mit dem Beatmungsgerät gekoppelt. Es kann auch wenigstens ein Computer bzw. Smartphone, Tablet-Computer o.ä. per Kabel bzw. drahtlos, z. B. WIFI, Bluetooth etc. mit dem Beatmungsgerät gekoppelt werden. Insbesondere umfasst das Speichermedium wenigstens einen spezifischen Datensatz, zum Beispiel ein Zertifikat, durch welchen der Berechtigungscode generiert und/oder abgerufen wird.

In einer vorteilhaften Ausgestaltung wird der angeforderte Berechtigungscode dem Benutzer auf wenigstens einer Anzeigeeinrichtung, insbesondere des Beatmungsgerätes, angezeigt. Die Anzeigeeinrichtung umfasst beispielsweise ein Display oder ist als solches ausgebildet. So kann zuverlässig erreicht werden, dass das Beatmungsgerät auch vor dem vorgesehenen Benutzer steht, wenn der Zugriff eingerichtet wird.

Möglich ist auch, dass der angeforderte Berechtigungscode auf wenigstens einem, insbesondere entnehmbaren, Speichermedium abgelegt wird. Dadurch kann der Benutzer besonders komfortabel den Berechtigungscode an der Gegenstelle hinterlegen und Eingabefehler werden vermieden.

Vorzugsweise umfasst die Benutzeraktion wenigstens eine Registrierung des Benutzers gegenüber der Datenverarbeitungseinrichtung und besonders bevorzugt gegenüber der Gegenstelle. Dadurch kann insbesondere nur von einem registrierten Benutzer der Berechtigungscode angefordert werden. Durch eine solche Registrierung wird ein besonders hohes Maß an Datensicherheit bzw. Datenschutz erreicht.

Insbesondere erfolgt im Rahmen der Registrierung wenigstens eine Authentifizierung des Benutzers gegenüber der Datenverarbeitungseinrichtung. Möglich ist auch, dass im Rahmen der Registrierung auch eine Autorisierung des Benutzers erfolgt. Bei der Autorisierung werden insbesondere die Zugriffsrechte auf bestimmte Therapiedaten für einen individuellen Benutzer festgelegt. Die Datenverarbeitungseinrichtung kann vorzugsweise mindestens zwei Berechtigungscodes von zwei unterschiedlichen Benutzern verwalten, die unterschiedliche Zugriffsrechte besitzen können.

Beispielsweise wird bei der Registrierung in der Gegenstelle wenigstens ein Benutzeraccount eingerichtet. Dabei wird insbesondere auch festgelegt, welche Art von Zugriffsrechten der Benutzer erhält. Beispielsweise kann ein Benutzeraccount für einen Betreuer oder Provider einen breiteren Zugriff auf Therapiedaten als ein Benutzeraccount für einen Patienten bieten.

Insbesondere umfasst die Benutzeraktion neben der Registrierung noch wenigstens einen weiteren Schritt, um den Berechtigungscode anfordern zu können. Möglich ist aber auch, dass die Vornahme der Registrierung bereits die Benutzeraktion darstellt, sodass mit der Registrierung auch zugleich der Berechtigungscode angefordert wird.

Bei der Registrierung wird dem Benutzer vorzugsweise wenigstens ein Zertifikat zugewiesen. Insbesondere muss das Zertifikat dem Beatmungsgerät vorgelegt werden, um den Berechtigungscode anfordern zu können. Möglich ist auch, dass das Zertifikat der Gegenstelle vorgelegt werden muss, um den Berechtigungscode anfordern zu können. Dadurch kann besonders zuverlässig sichergestellt werden, dass nur ein bestimmter Benutzer den Berechtigungscode anfordert. Beispielsweise umfasst das Zertifikat eine Identifikation des Benutzers. Insbesondere umfasst das Zertifikat eine Festlegung der Zugriffsrechte des Benutzers und/oder einer Autorisierung des Benutzers.

Insbesondere weist das Zertifikat eine zeitlich begrenzte Gültigkeit und/oder eine einmalige Gültigkeit auf. Möglich ist auch, dass die Gültigkeit des Zertifikats an den Benutzer gekoppelt ist. Das hat den Vorteil, dass beispielsweise bei einem Verlust eines Speichermediums mit einem darauf abgelegten Zertifikat das Zertifikat für ungültig erklärt werden kann, sodass ein Missbrauch des verlorenen Speichermediums verhindert wird.

Bevorzugt umfasst das Zertifikat eine Zuordnung des Benutzers zu einer Benutzergruppe. Insbesondere ist für jede Benutzergruppe eine bestimmte Autorisierung vorgesehen. Beispielsweise sind Ärzte bzw. Betreuer, Provider und Patienten unterschiedlichen Benutzergruppen mit verschiedenen Autorisierungen zugewiesen. Durch eine solche Ausgestaltung kann das Beatmungsgerät anhand des Zertifikates erkennen, wie weit dem jeweiligen Benutzer ein Zugriffsrecht eingeräumt werden darf.

Besonders bevorzugt wird das Zertifikat von der Gegenstelle auf einem transportablen Speichermedium abgelegt. Dabei kann der Berechtigungscode vorzugsweise nur angefordert werden, wenn das Beatmungsgerät mit dem Speichermedium gekoppelt ist und insbesondere einen Zugriff auf das Speichermedium hat.

Beispielsweise wird das Zertifikat auf dem Speichermedium abgelegt, wenn sich der Benutzer über die Gegenstelle an einem Server registriert. Der Benutzer kann das Speichermedium zum Beatmungsgerät mitnehmen und dort einlegen. Anhand des Zertifikats erkennt das Beatmungsgerät vorzugsweise den Benutzer und insbesondere auch seine Autorisierung und gibt vorzugsweise einen passenden Berechtigungscode aus bzw. überträgt den Berechtigungscode oder das Zertifikat zurück an die Gegenstelle, die insbesondere die Gültigkeit überprüft und den Benutzer identifiziert.

Das Zertifikat kann wenigstens ein das Beatmungsgerät identifizierendes Gerätezertifikat und/oder wenigstens ein den Benutzer identifizierendes Benutzerzertifikat umfassen oder als ein solches ausgebildet sein.

Bevorzugt wird von der Gegenstelle zusätzlich zu dem Zertifikat auch wenigstens ein eindeutig identifizierendes Gerätezertifikat auf das Speichermedium abgelegt. In einer solchen Ausgestaltung ist das Zertifikat insbesondere als ein Benutzerzertifikat ausgebildet. Insbesondere wird das Gerätezertifikat vom Beatmungssystem eingelesen und vorzugsweise wieder zurück an die Gegenstelle übertragen. Dadurch kann die Gegenstelle prüfen, dass es sich wirklich um ein legitimiertes Beatmungssystem handelt und nicht um eine Nachahmung, die Daten an die Gegenstelle versenden möchte, um dort verfälschte, ungültige oder erfundene Therapiedaten abzulegen.

Möglich ist aber auch, dass ein nicht-transportables bzw. festinstalliertes Speichermedium vorgesehen ist. Dann wird beispielsweise eine Netzwerkverbindung zwischen dem Speichermedium mit dem Zertifikat und dem Beatmungsgerät aufgebaut, um den Berechtigungscode anfordern zu können.

Besonders bevorzugt wird der Berechtigungscode wenigstens teilweise durch das Beatmungsgerät unter Einbeziehung des Zertifikats generiert. Das hat den Vorteil, dass ein Benutzer und beispielsweise seine Gruppenzugehörigkeit bzw. Autorisierung anhand des Berechtigungscodes identifiziert werden kann. Möglich ist auch, dass der Berechtigungscode wenigstens teilweise durch die Gegenstelle unter Einbeziehung des Zertifikats generiert wird.

Insbesondere umfasst der Berechtigungscode wenigstens einen Teil des Zertifikats und/oder wenigstens einen Teil eines Gerätecodes des Beatmungsgerätes. Der Gerätecode umfasst beispielsweise wenigstens eine Seriennummer und/oder wenigstens eine Geräteidentifikationsnummer. So kann auch das Beatmungsgerät besonders gut identifiziert werden, wenn es sich mit dem Berechtigungscode anmeldet. Möglich ist auch eine Generierung des Berechtigungscodes unabhängig von dem Zertifikat.

In einer besonders bevorzugten Ausgestaltung wird der Zugriff auf die Therapiedaten freigegeben, wenn sich der registrierte Benutzer bei der Gegenstelle anmeldet, nachdem er zuvor als ein registrierter Benutzer den Berechtigungscode angefordert hat. Dabei ist besonders bevorzugt, dass der Benutzer zuvor den Berechtigungscode unter Vorlage des Zertifikats angefordert hat. Eine solche Ausgestaltung ist besonders sicher und zugleich sehr komfortabel, da der Benutzer den Berechtigungscode nicht in der Gegenstelle eingeben muss.

Dabei erfolgt insbesondere eine Übertragung des Berechtigungscodes von dem Beatmungsgerät zu der Gegenstelle über wenigstens eine Netzwerkverbindung bzw. drahtlose Datenverbindung. Die Übertragung erfolgt insbesondere automatisch, wenn ein registrierter und vorzugsweise zertifizierter Benutzer den Berechtigungscode anfordert.

In einer vorteilhaften Ausgestaltung wird der Zugriff auf die Therapiedaten erst freigegeben, wenn der Benutzer den ihm zur Verfügung gestellten Berechtigungscode der Datenverarbeitungseinrichtung und insbesondere der Gegenstelle mitteilt. So kann besonders zuverlässig gewährleistet werden, dass die Person, die von der Gegenstelle aus auf die Therapiedaten zugreifen möchte, auch die Person ist, die das Beatmungsgerät besucht hat und den Berechtigungscode dort angefordert hat.

Vorzugsweise gleicht die Datenverarbeitungseinrichtung den vom Benutzer mitgeteilten Berechtigungscode mit einem generierten und/oder hinterlegten Berechtigungscode ab. Insbesondere wird der Zugriff auf die Therapiedaten nur freigegeben, wenn der Abgleich eine geeignete Übereinstimmung anzeigt.

Insbesondere wird der Berechtigungscode durch den Benutzer in der Gegenstelle eingegeben. Dadurch teilt der Benutzer den ihm zur Verfügung gestellten Berechtigungscode der Gegenstelle mit, sodass der Zugriff auf die Therapiedaten freigegeben werden kann. Beispielsweise notiert sich der Benutzer den Berechtigungscode, während er bei dem Patienten mit dem Beatmungsgerät ist. Anschließend baut er an seinem Arbeitsplatz eine Verbindung mit der Gegenstelle auf und gibt dort den Berechtigungscode ein.

Möglich ist aber auch, dass der Berechtigungscode über wenigstens eine Spracherkennung eingegeben wird. Die Übermittlung des Berechtigungscodes kann auch wenigstens einen anderen manuellen Schritt umfassen, beispielsweise den Transport des auf einem Speichermedium gespeicherten Berechtigungscodes vom Beatmungsgerät zur Gegenstelle. Die Eingabe kann auch dadurch erfolgen, dass der Benutzer die Gegenstelle mit wenigstens einem Speichermedium koppelt, auf dem er den Berechtigungscode gespeichert hat..Möglich ist auch eine optische Eingabe des Berechtigungscodes, beispielsweise als QR-Code, der insbesondere mit Hilfe einer Kamera ausgelesen und später ausgewertet wird, oder dergleichen.

In einer vorteilhaften Ausgestaltung übermittelt das Beatmungsgerät den Berechtigungscode wenigstens teilweise selbstständig an die Gegenstelle. Eine selbstständige Übermittlung ist besonders bevorzugt, wenn das Beatmungsgerät den Berechtigungscode wenigstens teilweise anhand eines Zertifikats generiert. So kann zuverlässig gewährleistet werden, dass eine Übermittlung nur bei einem berechtigten Benutzer erfolgt.

Insbesondere umfasst der Berechtigungscode wenigstens eine Seriennummer und/oder wenigstens eine Geräteidentifikationsnummer des Beatmungsgerätes. Möglich ist auch, dass der Berechtigungscode wenigstens teilweise aus einer Seriennummer und/oder Geräteidentifikationsnummer generiert wird. Der Berechtigungscode umfasst insbesondere wenigstens eine Ziffer und/oder wenigstens einen Buchstaben und/oder Sonderzeichen. Bevorzugt umfasst der Berechtigungscode wenigstens eine Abfolge von wenigstens vier Zeichen oder mehr. Der Berechtigungscode kann wenigstens teilweise zufällig generiert werden.

Der Berechtigungscode kann auch wenigstens eine definierte Abfolge von Lauten und/oder Gesten und/oder Bewegungen umfassen. Insbesondere umfasst der Berechtigungscode wenigstens eine Geste und/oder Bewegung wenigstens eines Fingers. Der Berechtigungscode kann auch wenigstens einen Fingerabdruck und/oder wenigstens ein Abbild wenigstens eines Teils der Iris und/oder der Netzhaut des Benutzers umfassen.

Insbesondere umfasst Berechtigungscode wenigstens eine Prüfstelle. Die Prüfstelle lässt sich insbesondere anhand der Seriennummer und/oder Geräteidentifikationsnummer des Beatmungsgerätes und/oder restlicher Stellen des Berechtigungscodes ermitteln. Das hat den Vorteil, dass vom Beatmungsgerät und/oder der Gegenstelle bereits eine Überprüfung auf Eingabefehler erfolgen kann, bevor der gesamte Berechtigungscode an das Beatmungsgerät bzw. die Gegenstelle übermittelt wird.

Bevorzugt umfasst der Berechtigungscode wenigstens eine Information über eine Autorisierung des Benutzers hinsichtlich des Zugriffs auf die Therapiedaten. Beispielsweise gibt der Berechtigungscode an, ob der Benutzer für einen Zugriff auf alle Therapiedaten oder nur für einen teilweisen Zugriff autorisiert ist. Der Berechtigungscode kann den Benutzer wenigstens einer Benutzergruppe zuordnen. Dies kann bevorzugt durch die Integration eines Benutzertyps in den Berechtigungscode sein, anhand dessen die Gegenstelle die Zugriffsrechte verwalten kann.

Der Berechtigungscode kann wenigstens eine Information über wenigstens ein Leserecht und/oder Schreibrecht des Benutzers umfassen. Vorzugsweise erfolgt eine Zuordnung von Lese- und Schreibrechten zu einem Benutzer im Rahmen der Registrierung des Benutzers und besonders bevorzugt anhand des Zertifikats des Benutzers. Beispielsweise umfasst das Zertifikat wenigstens ein Leserecht und/oder Schreibrecht.

Bevorzugt unterscheidet die Datenverarbeitungseinrichtung bzw. das Beatmungssystem bei den Zugriffsrechten auch zwischen Lese- und Schreibrechten. Leserechte werden insbesondere benötigt, um Therapiedaten des Beatmungssystems an der Gegenstelle auslesen zu können. Schreibrechte werden insbesondere benötigt, um Therapiedaten des Beatmungssystems an der Gegenstelle ändern zu können, z. B. die Beatmungseinstellungen, insbesondere mindestens einen Beatmungsdruck bzw. eine Druckgrenze, Beatmungsfrequenz oder eine Befeuchtungsleitung.

Besonders bevorzugt ist vorgesehen, dass an der Datenverarbeitungseinrichtung bzw. am Beatmungssystem selbst Schreibrechte über die Datenverarbeitungseinrichtung grundsätzlich blockiert werden können. Das erfolgt z. B. durch eine entsprechende Eingabe am User Interface, ein Sprachkommando oder eine Eingabe per Kabel bzw. Speichermedium. Dies bedeutet eine besonders große Sicherheit. Steht z. B. fest, dass eine Fernverstellung des Beatmungssystems durch legitimierte Benutzer der Gegenstelle ohnehin nicht vorgenommen werden soll, wird damit verhindert, dass nicht legitimierte Benutzer oder eine unbefugte Nachahmung der Gegenstelle eine Fernverstellung vornimmt.

In allen Ausgestaltungen ist es besonders bevorzugt, dass der Berechtigungscode wenigstens eine zeitlich begrenzte Gültigkeit und/oder eine einmalige Gültigkeit aufweist. Insbesondere endet mit Ablauf der Gültigkeit auch der berechtigte Zugriff auf die Therapiedaten. Der Berechtigungscode kann nur für einen einmaligen Zugriff gültig sein. Möglich ist auch, dass der Berechtigungscode nur gültig ist, um wenigstens einen Zugriff einzurichten. Der eingerichtete Zugriff kann dann auch längerfristig gültig sein, auch wenn der Berechtigungscode selbst nicht mehr gültig ist.

Möglich ist auch, dass der Berechtigungscode eine Gültigkeit besitzt, welche für die Dauer eines vorgesehenen Benutzungszeitraums des Beatmungssystems begrenzt ist. Dadurch kann besonders gut verhindert werden, dass unberechtigte Zugriffe auf nicht mehr benutzte Beatmungsgeräte stattfinden. Insbesondere muss dann bei einem Patientenwechsel wenigstens ein neuer Berechtigungscode angefordert werden.

Bevorzugt ist der Berechtigungscode nur für einen bestimmten Benutzer und insbesondere nur für einen registrierten Benutzer gültig. Möglich ist auch, dass der Berechtigungscode für eine Mehrzahl von Benutzern und beispielsweise für eine Benutzergruppe gültig ist.

Insbesondere ist die Gültigkeit des Berechtigungscodes an den Benutzer gekoppelt. Vorzugsweise ist die Gültigkeit des Berechtigungscodes an die Autorisierung des Benutzers gekoppelt. Die Koppelung erfolgt insbesondere über eine Zuordnung des Berechtigungscodes an eine Registrierung bzw. an ein Benutzerzertifikat. Beispielsweise ist möglich, dass die Gültigkeit eines Berechtigungscodes endet, wenn die Autorisierung des Benutzers beschränkt und/oder erweitert wird. So wird erreicht, dass die Zugriffsrechte, welche durch den Berechtigungscode freigegeben werden, auch der Autorisierung des Benutzers entsprechen. So kann verhindert werden, dass vertrauliche Therapiedaten nicht von Benutzern eingesehen werden, welche nur auf allgemeine Daten zugreifen dürfen.

Besonders bevorzugt wird die Gültigkeit des Berechtigungscodes und insbesondere auch des Zugriffs zumindest für bestimmte Benutzer aufgehoben, wenn das Beatmungsgerät für einen neuen Patienten eingesetzt wird. Beispielsweise überwacht die Datenverarbeitungseinrichtung dazu wenigstens einen charakteristischen Parameter für eine Zuordnung des Beatmungsgerätes an einen Patienten. Dieser Parameter kann beispielsweise ein Patientenname und/oder eine Patientenadresse sein. Dann verliert der Berechtigungscode beispielsweise seine Gültigkeit oder muss erneuert werden, wenn ein derartiger Parameter verändert wird. Ein Patientenwechsel kann von der Gegenstelle und dem Beatmungssystem bevorzugt auch indirekt erkannt werden, z. B. bei Löschen der Therapiedaten im Beatmungsgerät oder eine längere Pause in der Benutzung des Beatmungsgerätes, bevorzugt mehr als eine Woche.

Vorzugsweise ist der Berechtigungscode, welcher vom Benutzer angefordert wird, wenigstens teilweise in dem Beatmungsgerät hinterlegt und/oder wird wenigstens teilweise durch das Beatmungsgerät generiert. Dazu ist in dem Beatmungsgerät wenigstens eine Logik und/oder wenigstens ein Algorithmus hinterlegt. Insbesondere ist auch die Gegenstelle dazu geeignet und ausgebildet, den gleichen Berechtigungscode zu generieren, sodass ein Abgleich möglich ist. Beispielsweise ist dazu die gleiche Logik bzw. der gleiche Algorithmus sowohl in der Gegenstelle als auch im Beatmungsgerät hinterlegt. Der gleiche Berechtigungscode kann auch in der Gegenstelle hinterlegt sein, um einen Abgleich zu ermöglichen. Der Berechtigungscode kann auch über eine Netzwerkverbindung vom Beatmungsgerät zur Gegenstelle gesendet werden, um einen Abgleich zu ermöglichen.

Möglich ist auch, dass wenigstens ein Teil des Berechtigungscodes, der vom Benutzer angefordert wird, räumlich getrennt von dem Beatmungsgerät und vorzugsweise in der Gegenstelle generiert wird und/oder hinterlegt ist. Der Berechtigungscode kann auch wenigstens teilweise an einem vom Beatmungssystem räumlich getrennten Ort generiert werden, an dem sich wenigstens ein Code-Generator befindet. Beispielsweise sind die Datenverarbeitungseinrichtung und vorzugsweise die Gegenstelle dazu über wenigstens eine Netzwerkverbindung mit einem Code-Generator verbunden. Der Berechtigungscode kann dann an das Beatmungsgerät übertragen werden, beispielsweise über eine Netzwerkverbindung oder auch durch den Transport eines Speichermediums oder auch durch Programmierung während der Fertigung, z. B. über einen Prüfstand.

Das erfindungsgemäße Beatmungssystem umfasst wenigstens ein Beatmungsgerät mit wenigstens einer Beatmungseinrichtung zur Erzeugung wenigstens eines Luftstroms für eine Atemtherapie. Das Beatmungssystem umfasst wenigstens eine räumlich von dem Beatmungsgerät getrennte Gegenstelle und wenigstens eine Datenverarbeitungseinrichtung. Mit der Datenverarbeitungseinrichtung sind Therapiedaten zwischen dem Beatmungsgerät und der Gegenstelle übertragbar. In der Datenverarbeitungseinrichtung ist wenigstens ein Berechtigungscode hinterlegbar, um einen Benutzer über die Gegenstelle einen berechtigten Zugriff auf die Therapiedaten zu gewähren. Dabei ist die Datenverarbeitungseinrichtung dazu geeignet und ausgebildet, dem Benutzer den Berechtigungscode zur Verfügung zu stellen, wenn dieser den Berechtigungscode durch wenigstens eine gegenüber der Datenverarbeitungseinrichtung vorgenommene Benutzeraktion anfordert.

Auch das erfindungsgemäße Beatmungssystem bietet einen verbesserten Zugriff auf die Therapiedaten, sodass ein hohes Maß an Datensicherheit bzw. Datenschutz erreicht wird.

Besonders bevorzugt ist das Beatmungssystem dazu geeignet und ausgebildet, nach dem erfindungsgemäßen Verfahren betrieben zu werden. Die Datenverarbeitungseinrichtung umfasst insbesondere wenigstens eine Benutzerschnittstelle. Die Benutzerschnittstelle ist insbesondere dazu geeignet und ausgebildet, wenigstens eine Eingabe des Benutzers aufzunehmen und in ein elektronisch zu verarbeitendes Signal umzusetzen. Die Benutzerschnittstelle umfasst beispielsweise wenigstens ein Human-Interface-Device. Beispielsweise kann über die Benutzerschnittstelle wenigstens eine Texteingabe und/oder Spracheingabe erfolgen. Mit der Benutzerschnittstelle kann auch eine biometrische Erfassung des Benutzers durchführbar sein.

Die Datenverarbeitungseinrichtung ist insbesondere dazu geeignet und ausgebildet, das Beatmungsgerät und die Gegenstelle über wenigstens eine Netzwerkverbindung miteinander zu verbinden. Dabei kann die Datenverarbeitungseinrichtung wenigstens teilweise in dem Beatmungsgerät und/oder der Gegenstelle integriert sein bzw. wenigstens teilweise durch diese bereitgestellt werden. Die Datenverarbeitungseinrichtung kann das Beatmungsgerät und/oder die Gegenstelle wenigstens teilweise umfassen. Die Datenverarbeitungseinrichtung ist insbesondere dazu geeignet und ausgebildet, wenigstens eine Speichereinrichtung des Beatmungsgeräts und/oder der Gegenstelle auszulesen und/oder zu beschreiben.

Die Gegenstelle kann durch wenigstens ein Netzwerkgerät bereitgestellt werden oder wenigstens ein solches umfassen. Beispielsweise umfasst die Gegenstelle wenigstens einen Server und/oder wenigstens eine Rechnerwolke (Cloud).

Die Netzwerkverbindung nutzt insbesondere eine kabelgebundene Verbindung und/oder eine Mobilfunkverbindung und/oder das Internet, VPN, WLAN (Wireless Local Area Network) und/oder LAN (Local Area Network). Dazu sind an dem Beatmungsgerät und der Gegenstelle vorzugsweise entsprechende Schnittstellen vorgesehen.

Weitere Vorteile und Merkmale der vorliegenden Erfindung ergeben sich aus der Beschreibung der Ausführungsbeispiele, welche mit Bezug auf die beiliegende Figur im Folgenden erläutert werden.

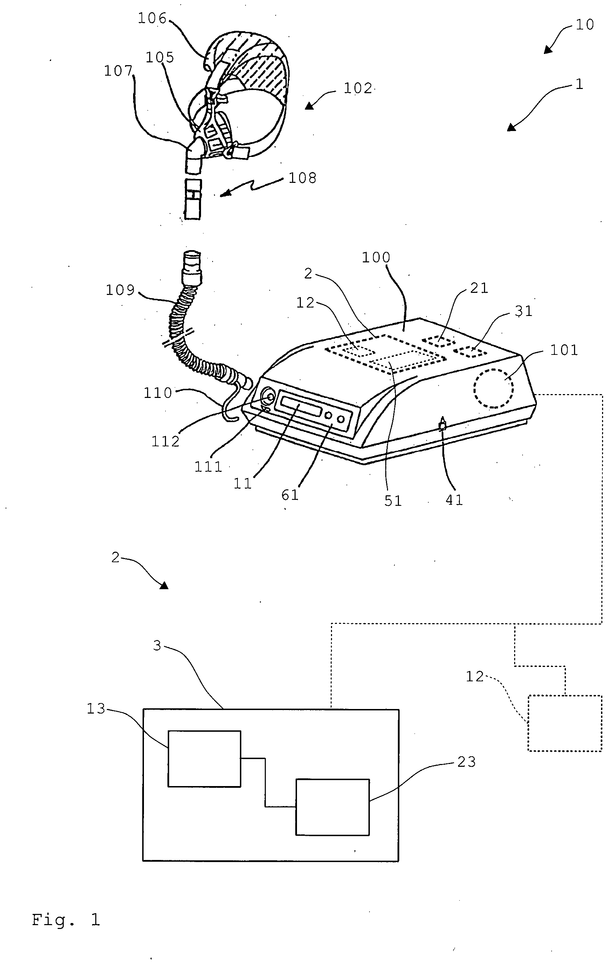

Die Figur 1 zeigt eine stark schematische Darstellung eines erfindungsgemäßen Beatmungssystems.

In der Figur 1 ist ein erfindungsgemäßes Beatmungssystem 10 gezeigt, welches hier ein als Heimbeatmungsgerät 11 bzw. Schlaftherapiegerät eingesetztes Beatmungsgerät 1 umfasst. Das Beatmungsgerät 1 kann aber auch als klinisches Beatmungsgerät 1 ausgebildet sein. Das Beatmungsgerät 1 ist zur Durchführung des erfindungsgemäßen Verfahrens geeignet und ausgebildet.

Das Beatmungsgerät 1 umfasst eine Beatmungseinrichtung 100 mit einer Gebläseeinrichtung 101 zur Erzeugung eines Luftstromes für die Beatmung. Zur Steuerung der Beatmungseinrichtung 100 und zur Erfassung von Therapiedaten ist hier eine Überwachungseinrichtung 21 vorgesehen. Die Bedienung und Einstellung des Beatmungsgerätes 1 erfolgt über eine Benutzerschnittstelle 61 mit Bedienelementen 103 und einer Anzeigeeinrichtung 11.

Das Beatmungsgerät 1 weist eine Atemschnittstelle 102 auf, um den Luftstrom einem Benutzer zur Beatmung zuzuführen. Die hier gezeigte Atemschnittstelle 102 ist eine als Nasalmaske ausgebildete Atemmaske 105. Zur Fixierung der Atemmaske 105 ist eine Kopfhaube 106 vorgesehen. Die Atemschnittstelle 102 kann beispielsweise auch als eine Vollgesichtsmaske, als ein Nasal-Pillow, als ein Tubus oder als eine Larynxmaske ausgestaltet sein.

Zur Verbindung der Atemschnittstelle 102 mit der Beatmungseinrichtung 100 ist ein Verbindungsschlauch 109 vorgesehen, der mittels einer Koppeleinrichtung 112 mit der Beatmungseinrichtung 100 verbunden wird. Über ein Kuppelungselement 107 wird der Verbindungsschlauch 109 mit der Atemschnittstelle 102 verbunden. Zwischen dem Verbindungsschlauch 109 und dem Kuppelungselement 107 ist ein Ausatmungselement 108 angeordnet, welches ein Ventil umfasst oder als ein solches ausgebildet ist. Das Ausatmungselement 108 ist insbesondere dazu vorgesehen, während der Ausatmung des Benutzers ein Rückatmen in das Beatmungsgerät 1 zu verhindern.

Die Überwachungseinrichtung 21 ist hier mit einer nicht näher dargestellten Sensoreinrichtung wirkverbunden, welche einen oder mehrere Sensoren zur Erfassung von Geräteparametern und/oder Patientenparametern und/oder anderer für die Beatmung charakteristischer Größen aufweist.

Zum Beispiel umfasst die Überwachungseinrichtung 21 einen hier nicht näher gezeigten Drucksensor, welcher die Druckverhältnisse bezüglich der Atemschnittstelle 102 erfasst. Dazu ist der Drucksensor über einen Druckmessschlauch 110 mit der Atemschnittstelle 102 verbunden. Über einen Eingangsstutzen 111 ist der Druckmessschlauch 110 an die Überwachungseinrichtung 21 angebunden.

Des Weiteren dient die Überwachungseinrichtung 21 hier zur Ansteuerung der Gebläseeinrichtung 101. Die Überwachungseinrichtung 21 stellt einen notwendigen Minimaldruck bereit und kompensiert Druckschwankungen, die durch die Atemtätigkeit des Benutzers bedingt sind. Beispielsweise erfasst die Überwachungseinrichtung 21 auch den gegenwärtigen Druck in der Atemmaske 105 und regelt die Leistung der Gebläseeinrichtung 101 entsprechend nach, bis ein gewünschter Beatmungsdruck anliegt.

Die zur Einstellung der Beatmungseinrichtung 100 bzw. der Gebläseeinrichtung 101 benötigten Geräteparameter sowie die Gerätekonfiguration und/oder Gerätesoftware sind in einer Speichereinrichtung 31 hinterlegt.

Die-Überwachungseinrichtung 21 kann hier auch zur Erfassung von Patientenparametern ausgebildet sein. Die Überwachungseinrichtung 21 kann dazu mit Sensoren zur Messung der Atemexkursion, zur Messung einer Sauerstoffsättigung des Blutes und/oder zur Messung einer EEG-, einer EMG-, einer EOG- oder einer EKG-Aktivität ausgestattet sein.

Das hier gezeigte Beatmungsgerät 1 kann als ein Fix-Level-Gerät oder auch als ein Automatic-Level-Gerät ausgebildet sein. Insbesondere erfolgt dabei durch die Überwachungseinrichtung 21 eine Regelung auf Soll-Geräteparameter, welche zuvor anhand der charakteristischen Atmung eines Benutzers individuell berechnet und festgelegt worden sind.

Es ist auch möglich, das die Beatmungseinrichtung 100 dynamisch und insbesondere je nach Atemphase des Benutzers angepasst wird. Beispielsweise kann anhand der Überwachungseinrichtung 21 ein Atemphasenwechsel erkannt werden, sodass je nach Atemphase ein höherer bzw. niedrigerer Druck bereitgestellt werden kann. Beispielsweise kann das Beatmungsgerät 1 als ein CPAP- oder APAP-Gerät ausgebildet sein. Das Beatmungsgerät 1 kann auch als ein Bilevel-Gerät ausgebildet sein. Beispielsweise reagiert das Beatmungsgerät 1 auf bestimmte Atemereignisse, wie z. B. Schnarchen, Atemabflachungen und/oder obstruktive Druckspitzen mit entsprechenden Einstellungen der Geräteparameter.

Die von der Überwachungseinrichtung 21 erfassten Druckverhältnisse werden zusammen mit weiteren Geräteparametern in einer Speichereinrichtung 31 hinterlegt. Zudem werden die von der Überwachungseinrichtung 21 eingestellten Druckverhältnisse bzw. die vorgenommenen Druckanpassungen ebenfalls als Geräteparameter in der Speichereinrichtung 31 hinterlegt. Auch die erfassten Patientenparameter können in der der Speichereinrichtung 31 hinterlegt werden. Zudem können Daten über Compliance und Maskendichtigkeit gespeichert werden.

Als Geräteparameter können beispielsweise ein Start-Therapie-Druck, ein maximaler Therapie-Druck, ein minimaler Therapie-Druck und/oder ein Zielvolumen und/oder andere zur Einstellung der Beatmungseinrichtung 100 geeignete Geräteparameter hinterlegt sein. Diese Geräteparameter werden von der Überwachungseinrichtung 21 zur Einstellung der Beatmungseinrichtung 100 aus der Speichereinrichtung 31 abgerufen.

Des Weiteren werden die über den Therapiezeitraum erfassten Druckverhältnisse und/oder sonstigen Geräteparameter und/oder Patientenparameter im Rahmen von Therapieverläufen in der Speichereinrichtung 31 hinterlegt. Als Therapieverläufe können z. B. ein Flow-Verlauf, ein Druck-Verlauf und/oder ein Event-Verlauf registriert werden. Die Therapieverläufe werden der Speichereinrichtung 31 von der Überwachungseinrichtung 21 bereitgestellt, die diese Daten während der Therapie erfasst.

Die in der Speichereinrichtung 31 hinterlegten Geräteparameter und/oder Patientenparameter und/oder Therapieverläufe werden abgerufen und zu einer oder mehrerer Therapiestatistiken ausgewertet. Die Therapiestatistik wird in der Speichereinrichtung 31 hinterlegt. Dabei können z. B. ein mittlerer Druck und/oder die Therapiedauer und/oder ein Leckageparameter ermittelt und abgespeichert werden.

Die Therapiestatistik kann auch als Statistik für jeweils eine Therapieperiode ausgestaltet sein, welche mehrere Therapiebehandlungen umfasst. Die Therapiestatistik kann auch eine Auswertung der Mitwirkungsbereitschaft des Benutzers umfassen. Die wie zuvor beschrieben in der Speichereinrichtung 31 abgelegten Werte und Größen werden im Rahmen der vorliegenden Erfindung als Therapiedaten bzw. Daten bezeichnet.

Um die Therapiedaten einer therapeutischen bzw. diagnostischen Analyse unterziehen zu können oder um die Funktion des Beatmungsgerätes 1 überwachen zu können, ist hier eine Übertragung der Therapiedaten an wenigstens eine Gegenstelle 3 mittels einer Datenverarbeitungseinrichtung 2 vorgesehen. Die Datenverarbeitungseinrichtung 2 stellt hier die Komponenten bzw. Software bereit, damit die Gegenstelle 3 die Daten des Beatmungsgerätes 1 interpretieren kann und umgekehrt.

Das Beatmungssystem 10 kann auch zwei oder mehr Gegenstellen 3 umfassen, welche mit einem oder auch mehreren Beatmungsgeräten 1 verbunden sind.

Die Übertragung erfolgt mittels einer Übertragungseinrichtung 51 drahtlos und/oder drahtgebunden. Die Übertragung kann über eine oder mehrere Kabel-Schnittstellen erfolgen, z. B. USB, seriell, LAN, Datenbus etc. Die Übertragung kann auch über eine oder mehrere drahtlose Schnittstellen erfolgen, z. B. Mobilfunk, LPWAN, Bluetooth, Infrarot, Sigfox, Lora etc.

Die Gegenstelle 3 kann wenigstens einen Server 13 und/oder wenigstens einen Personal Computer (PC) 23 umfassen oder auch als eine Rechnerwolke bzw. Cloud ausgebildet sein. So kann z. B. von einem PC 23 aus auf einen Webserver 13 zugegriffen werden, welcher wiederum über eine Netzwerkverbindung mit einem oder mehreren Beatmungsgeräten 1 verbunden ist. Der Datenaustausch wird dabei von der Datenverarbeitungseinrichtung 2 überwacht bzw. gesteuert.

Zudem können die Therapiedaten auch wenigstens teilweise auf einem transportablen Speichermedium 41 abgelegt werden. Das Speichermedium 41 ist beispielsweise als eine Speicherkarte oder eine Festplatte oder ein USB-Massenspeicher ausgebildet. Das Speichermedium 41 kann aus dem Gerät entnommen und über ein Lesegerät und z. B. einen Computer, Tablet-Computer, Smartphone etc. ausgelesen werden.

Die Therapiedaten können auch über ein im Gerät 1 befindliches und/oder an das Gerät 1 angeschlossenes Display 11 bzw. eine Benutzerschnittstelle 61 ausgelesen werden.

Über dieselben Schnittstellen kann das Gerät 1 mit neuen Konfigurationsdaten oder neuem Programmcode ausgestattet oder Funktionen im Gerät 1 aktiviert werden. Ein oder mehrere Datenspeicher 31, 41 im Gerät 1 werden also bevorzugt von außen beschrieben und/oder gelesen.

Von der Gegenstelle 3 aus kann ein Zugriff auf die Therapiedaten von einem entfernten Ort erfolgen, sodass eine ortsunabhängige Auswertung möglich ist. So können Ärzte, Provider, Patienten und andere auf die Daten im Beatmungssystem 10 zugreifen. Der Zugriff wird aus Datenschutz und Datensicherheitsgründen vorzugsweise auf die selbst eingesetzten Geräte 1 beschränkt. Der Patient bekommt z. B. nur auf sein Gerät 1 Zugriff. Fachpersonal wie Provider und Ärzte bekommen z. B. auf die Geräte 1 aller ihrer Patienten Zugriff.

Dabei kann jeder Nutzer den Zugriff zu seinen Geräten 1 selbst einrichten. Oder einer der Nutzer richtet den Zugriff ein und gibt die Berechtigungen dann innerhalb der Datenverarbeitungseinrichtung 2 bzw. des Beatmungssystems 10 und vorzugsweise der Gegenstelle 3 an andere Nutzer weiter. Es muss also in der Datenverarbeitungseinrichtung 2 eingegeben werden, dass mindestens ein Nutzer auf ein bestimmtes Gerät 1 zugreifen möchte bzw. darf.

Um den Datenverkehr in Bezug auf Datenschutz und Datensicherheit besonders sicher zu gestalten, ist der Zugriff hier nur unter Anwendung eines Berechtigungscodes möglich. Dabei wird der Berechtigungscode dem Benutzer erst dann zur Verfügung gestellt, wenn dieser den Berechtigungscode anfordert. Dadurch kann der Code z. B. nicht einfach von Unberechtigten an der Geräteaußenseite abgelesen werden. Zur Anforderung des Berechtigungscodes muss wenigstens eine Benutzeraktion gegenüber der Datenverarbeitungseinrichtung 2 vorgenommen werden.

Das Abrufen des Berechtigungscodes erfolgt hier z. B. auf dem Display 11 bzw. Userinterface 61 des Beatmungsgerätes 1 oder auf einem extern angeschlossenen Display oder per Sprachausgabe oder auf einem entnehmbaren Speichermedium 41. Der Berechtigungscode ist hier nur in bestimmten Betriebszuständen des Gerätes 1 am Gerät 1 abrufbar.

Der Betriebszustand, in dem der Berechtigungscode abgerufen wird, wird hier durch eine Benutzeraktion hergestellt. Beispielsweise Drücken mindestens einer Taste / eines Drehknopfes / eines Touchscreenbuttons am Gerät 1 oder eine Kombination aus Tasten bzw. anderen Bedienelementen; Eingabe eines Sprachkommandos; Eingabe eines Speichermediums 41 mit einem bestimmten Code, anhand dessen das Gerät 1 den Betriebszustand herstellt; Eingabe eines Kommandos an das Gerät 1 über eine der kabelgebundenen oder drahtlosen Schnittstellen; Überprüfung eines Fingerabdruckes oder Abbildes der Iris bzw. Netzhaut eines Benutzers.

In einer Ausgestaltung ist der Betriebszustand zeitlich befristet. Der Berechtigungscode kann also nicht beliebig lange Zeit am Gerät 1 abgelesen bzw. ausgelesen werden. Eine bevorzugte zeitliche Befristung beträgt zwischen 5 Sekunden und 5 Minuten. Möglich sind auch andere Zeiträume. Bevorzugt kann der Betriebszustand vom Benutzer vorzeitig beendet werden.

Das Gerät 1 umfasst hier eine Logik, den Berechtigungscode anhand seiner Seriennummer oder Geräte-Identifikationscode zu erzeugen oder abzurufen. Dieselbe Logik des Erzeugens oder Abrufens besitzt die Gegenstelle 3, die Gerätedaten auslesen bzw. anzeigen und Datenspeicher 31 des Gerätes 1 von außen beschreiben kann. So kann die Gegenstelle 3 prüfen, ob der Berechtigungscode korrekt ist.

In einer alternativen Ausgestaltung kann der Berechtigungscode von einem Code-Generator 12 erzeugt werden, der wiederum den Berechtigungscode sowohl dem Gerät 1 als auch der Gegenstelle 3 mitteilt. Damit kann das Gerät 1 den korrekten Berechtigungscode ausgeben und die Gegenstelle 3 die Korrektheit des Berechtigungscodes prüfen. Der Code-Generator 12 kann z. B. wenigstens einen Zufallsgenerator umfassen.

In der hier gezeigten Ausgestaltung ist der Code-Generator 12 Teil des Beatmungsgerätes 1. Der Code-Generator 12 kann aber auch Teil der Gegenstelle 3 sein. In einer anderen Ausgestaltung kann der Code-Generator 12 auch über eine Netzwerkverbindung mit dem Beatmungsgerät 1 und der Gegenstelle 3 verbunden sein, z. B. so wie hier gestrichelt dargestellt.

Dabei kann der Benutzer auch auf separatem Wege über den korrekten Berechtigungscode informiert werden. Besonders bevorzugt erlaubt das Beatmungsgerät 1 in diesem Fall die Abfrage medizinischer oder technischer Daten an einer eingebauten oder angeschlossenen Anzeigeeinheit 11 auch nur dann, wenn der korrekte Berechtigungscode am Gerät 1 eingegeben wurde.

Besonders bevorzugt wird der Berechtigungscode in einer verschlüsselten oder verschleierten Weise bei der Datenkommunikation zwischen der Gegenstelle 3 und dem Beatmungsgerät 1 mit übertragen. Eine Übertragung von der Gegenstelle 3 hin zum Beatmungsgerät 1 dient dazu, dass das Gerät 1 den korrekten Berechtigungscode ausgeben kann, falls die Gegenstelle 3 den Berechtigungscode zeitlich gesehen vor dem Beatmungsgerät 1 kennen sollte. Eine Übertragung vom Beatmungsgerät 1 hin zur Gegenstelle 3 dient dazu, dass die Gegenstelle 3 den korrekten Berechtigungscode überprüfen kann, falls das Beatmungsgerät 1 den Berechtigungscode zeitlich gesehen vor der Gegenstelle 3 kennen sollte.

In einer Ausführung der Erfindung gibt der Benutzer den angeforderten und bereitgestellten Berechtigungscode dann an der Gegenstelle 3 ein. Das erfolgt z. B. über Bedienelemente 103 und/oder über eine Spracheingabe und/oder anhand eines Speichermediums 41, dass mit der Gegenstelle 3 gekoppelt wird.

Nur bei Vorliegen eines gültigen Berechtigungscodes ermöglicht die Gegenstelle 3 eine Datenkommunikation bzw. das Abspeichern bzw. das Anzeigen bzw. das Verändern von Therapiedaten. So wird verhindert, dass aus Versehen oder absichtlich Daten des falschen Gerätes 1 angezeigt oder verändert werden.

Wird z. B. mehrfach der falsche Berechtigungscode eingegeben, kann in der Gegenstelle 3 der Zugriff auf das Beatmungsgerät 1 für einen Zeitraum von wenigstens einer Minute gesperrt werden. Außerdem wird bevorzugt eine Benachrichtigung versandt, z. B. an andere Nutzer oder Administratoren der Gegenstelle 3 oder an das Beatmungsgerät 1.

Der Zugriff kann mit einer Datenbank von infrage kommenden' Geräten abgeglichen werden, z. B. mit einem ERP-System. Bevorzugt wird geprüft, ob ein Provider das Gerät 1 erworben hat.

Bevorzugt unterscheidet die Gegenstelle 3 dabei verschiedene Benutzer und ermöglicht die Kommunikation bzw. das Speichern bzw. das Anzeigen bzw. das Verändern von Therapiedaten nur für diejenigen Benutzer, die den Berechtigungscode korrekt eingegeben oder eingelesen haben.

Besonders bevorzugt werden mehrere unterscheidbare Berechtigungscodes erzeugt, die z. B. in unterschiedlichen Betriebszuständen des Gerätes 1 ausgegeben werden oder verschiedenen Benutzern vom Code-Generator 12 mitgeteilt werden. Je nach Berechtigungscode kann somit die Rolle des Benutzers erkannt und die Zugriffsrechte auf eine bestimmte Teilmenge der zur Verfügung stehenden Therapiedaten begrenzt werden.

Beispielsweise können ein Patientencode und ein unterscheidbarer Ärztecode und/oder Providercode vorgesehen sein. Bei einem Zugriff auf Gerätedaten, z. B. über einen Webserver als Gegenstelle 3, bekommt der Patient eine Teilmenge der gespeicherten Daten in einer besonders für ihn aufbereiteten Ansicht mit für ihn optimierten Erklärungen präsentiert. Außerdem kann er über die Gegenstelle 3 nur bestimmte Konfigurationsparameter des Beatmungsgerätes 1 verändern, z. B. Komfortparameter.

Mit dem Ärztecode wird eine andere Teilmenge oder die Gesamtmenge der gespeicherten Therapiedaten in einer besonders für Ärzte aufbereiteten Ansicht dargestellt. Außerdem kann der Arzt über die Gegenstelle 3 auch therapeutisch relevante Konfigurationsparameter verändern, z. B. wenigstens einen Therapiedruck.

Der Berechtigungscode ist insbesondere nur für eine Auswahl von Beatmungsgeräten 1 gültig, bevorzugt nur für ein einzelnes Gerät 1 mit einer bestimmten Seriennummer bzw. Geräte-Identifikationscode.

In einer besonders bevorzugten Ausführungsform ist der Berechtigungscode zeitlich veränderlich. Dies bedeutet, dass es für das Beatmungsgerät 1 eine Vielzahl von Berechtigungscodes gibt bzw. generierbar sind.

Für den aktuellen Zugriff auf das Gerät 1 oder die von ihm empfangenen Therapiedaten sind jedoch nur wenige dieser Berechtigungscodes gültig, vorzugsweise nur ein einziger. Ein vorab notierter Code hat dann keine Gültigkeit mehr. Z. B. wird bei jedem Patienten- oder Besitzerwechsel ein neuer Berechtigungscode durch das Gerät 1 erzeugt.

Der zeitlich veränderliche Code wird z. B. in der Gegenstelle 3 erstellt und an das Gerät 1 übertragen. Damit kann zuverlässig gewährleistet werden, dass das Gerät 1 im Moment des Einrichtens eines Zugriffes bzw. während der Anforderung des Berechtigungscodes vor dem zukünftigen Nutzer steht.

Bei zeitlich veränderlichen Berechtigungscodes sind die folgenden Ausgestaltungen besonders vorteilhaft.

Zum Beispiel ist ein Berechtigungscode für einen längeren Zeitraum gültig, der durch ein Ereignis beendet wird. Danach ist ein neuer Berechtigungscode gültig. Mögliche Ereignisse bzw. Parameter zur Änderung des gültigen Codes können sein: Ablauf eines definierten Gültigkeitszeitraumes; Löschen von Gerätedaten; Zuordnung des Gerätes 1 zu einem neuen Patienten; Zuordnung des Gerätes 1 zu einer neuen Organisation, z. B. Arzt oder Betreiber oder Homecare-Provider; Ablauf eines definierten Zeitraumes, in dem nicht auf die Daten zugegriffen wurde; Zugriff eines neuen oder zusätzlichen Benutzers auf das Gerät 1 oder die von ihm empfangenen Daten; maßgebliche Veränderungen am Gerät 1, wie z. B. Austausch von Komponenten, Änderung von Therapieeinstellungen, Update von Programmcode im Gerät 1; Verwendung einer neuen oder zusätzlichen Datenschnittstelle oder Ausgabevorrichtung oder Anzeigevorrichtung oder einer Datenübertragungsart zwischen Gerät 1 und Gegenstelle 3; Maßgebliche Veränderungen an der Gegenstelle 3, wie z. B. Änderung von Programmcode, Änderung des Ortes, Änderung von Elektronikkomponenten.

In einer anderen Ausgestaltung wird für bestimmte Aktionen grundsätzlich jedes Mal ein neuer Berechtigungscode in Form eines Einmalcodes verlangt. Solche Aktionen bzw. Parameter sind beispielsweise: Zuordnung eines Patienten zu einem Gerät 1; Änderung von Konfigurationsparametern eines Gerätes 1; Aktualisierung des Programmcodes eines Gerätes 1; Auslesen von Datenmengen, die über das regelmäßig übertragene Maß hinaus gehen und die daher erhöhte Übertragungskosten verursachen; Übertragung von identifizierenden Therapiedaten; Zuordnung eines Patienten zu einer Organisation, z. B. betreuendem Arzt oder Provider; Löschen von Daten oder Patientenakten.

In einer möglichen Ausführungsform wird das Beatmungsgerät 1 erst durch Aktualisierung des Programmcodes in die Lage versetzt, Berechtigungscodes erzeugen bzw. empfangen bzw. Speichern bzw. ausgeben zu können.

Das bietet erhebliche Vorteile, insbesondere gegenüber dauerhaft in Form eines Aufklebers von außen auf das Gerät 1 aufgebrachte Codierungsmöglichkeiten. So wird ermöglicht, dass Geräte 1 zunächst ohne die Funktion der Verwaltung von Berechtigungscodes erzeugt und verwendet werden können. Erst nachträglich bei Bedarf wird diese Funktionalität ins Gerät 1 gebracht.

In einer besonders vorteilhaften Ausführungsform wird zusätzlich anhand eines insbesondere dauerhaft im Gerät 1 hinterlegten Codes (z. B. ein Zertifikat) und eines insbesondere dauerhaft in der der Gegenstelle 3 hinterlegten Codes (z. B. ein weiteres Zertifikat) zunächst geprüft, ob Beatmungsgerät 1 und Gegenstelle 3 wirklich authentische Produkte sind und keine Nachbildungen, um den Berechtigungscode zu umgehen bzw. diesen abzuhören. Bevor eine Datenkommunikation zwischen Beatmungsgerät 1 und Gegenstelle 3 erfolgt, überprüfen diese gegenseitig ihre Zertifikate oder kommunizieren beide mit einer zusätzlichen Prüfstelle, z. B. einem Zertifikate-Server, der die Echtheit der Zertifikate bestätigt.

In einer besonders vorteilhaften Ausführung umfasst die Benutzeraktion wenigstens eine Registrierung des Benutzers gegenüber der Gegenstelle 3. Dabei kann der Berechtigungscode nur von einem registrierten Benutzer angefordert werden.

Für die Registrierung richtet sich der Benutzer z. B. zunächst einen gesicherten Account auf einem bereitgestellten Server 13 ein, bei dem er sich authentifiziert. Ist der Benutzer am Server 13 angemeldet, kann er sich ein Speichermedium 41 bzw. Datenträger (z. B. eine SD-Karte) erstellen mit Daten, die ihn als Benutzer eindeutig identifizieren. Dazu speichert der Server 13 ein Benutzerzertifikat auf das Speichermedium 41, das dadurch zur "Key-Karte" wird. Das Zertifikat besteht insbesondere aus wenigstens einem Code, der den Benutzer identifiziert und z. B. über eine Checksumme gesichert ist.

Optional kann das Zertifikat bzw. der Code weitere Informationen beinhalten. Z. B. ein Attribut der Nutzergruppe: Ist der Benutzer Arzt, Patient oder Provider?

Das Zertifikat kann auch wenigstens einen einmaligen Code aufweisen, damit eine Key-Karte z. B. bei Verlust am Server 13 ungültig erklärt und eine neue, unterscheidbare Key-Karte erzeugt werden kann.

Das Zertifikat kann wenigstens ein Attribut enthalten, ob die Key-Karte nur für ein Gerät 1 gültig sein soll oder dauerhaft.

Das Zertifikat kann wenigstens eine Serveradresse bzw. eine Einwahl-Adresse ins Internet umfassen, die genau zu der Datenbank führt, auf die der jeweilige Besitzer der Key-Karte seinen Account besitzt.

Der Benutzer steckt die Key-Karte beim Patienten bei der Inbetriebnahme oder dem Moment der Tele-Initiierung in das Beatmungsgerät 1. Das Beatmungsgerät 1 erkennt das User-Zertifikat auf der Key-Karte und sendet eine Nachricht an die Gegenstelle 3 bzw. den Server. Diese Nachricht beinhaltet wenigstens einen Teil des User-Zertifikats, der den Benutzer identifiziert, und einen Code, der das Gerät identifiziert, z. B. eine Seriennummer und/oder eine Geräteidentifikationsnummer.

Der Server prüft dann die Gültigkeit beider Identitäten (User-Zertifikat und Geräte-Identifikation). Sind beide gültig, so werden Gerät 1 und Benutzer miteinander assoziiert, ohne dass irgendein Code manuell eingegeben werden muss. Der Benutzer findet das Gerät 1 beim nächsten Einloggen an der Gegenstelle 3 bzw. am Server und kann die Therapiedaten des Gerätes 1 ansehen bzw. dem Gerät 1 einen Patienten zuweisen.

Es kann vorgesehen sein, dass auch weitere Benutzer bzw. weitere Gegenstellen 3 auf das Gerät 1 zugreifen können. Entweder automatisch, z. B. eine Nutzergruppe, zu der der Benutzer gehört, der die Key-Karte ins Gerät 1 gesteckt hat, oder andere Nutzer, z. B. Ärzte, die der ursprüngliche Benutzer manuell für den Patienten auswählt.

Das Gerät 1 entscheidet vorzugsweise anhand der Attribute des User-Zertifikates, ob es dieses anschließend löscht bzw. entwertet oder unverändert für weitere Geräte auf der Key-Karte belässt.

Der Benutzer kann auch der Patient sein, der sich seine eigenen Therapiedaten über die Gegenstelle 3 bzw. am Server 13 ansehen will. Er kann genauso über eine Key-Karte den Zugriff auf die Daten seines Gerätes 1 freischalten.

Die Datenverarbeitungseinrichtung 2 bzw. der Server 13 der Gegenstelle 3 entscheidet vorzugsweise, ob er, getriggert durch ein bestimmtes Ereignis, ein neues Zertifikat verlangt, vor allem vom Patienten. Insbesondere werden dabei Ereignisse berücksichtigt, die auf einen Patientenwechsel hindeuten. Solche Ereignisse können z. B. anhand von charakteristischen Parametern erkannt werden. Solche Parameter sind z. B. im Gerät 1 gelöschte Therapiedaten, eine längere Nicht-Verwendung des Geräts 1, eine Zuweisung des Geräts 1 auf dem Server zu einem neuen Patienten.

Die hier vorgestellte Erfindung ermöglicht einen besonders sicheren Zugriff auf Therapiedaten und bietet zuverlässigen Schutz davor, dass es zu Verwechslungen kommt und dass das falsche Gerät 1 ausgelesen oder beschrieben wird. Zudem bietet es Schutz davor, dass sich Personen bewusst Zugang zum Auslesen oder Beschreiben von Beatmungsgeräten 1 verschaffen, für die sie keine Befugnis besitzen. Es kann auch wirkungsvoll entgegen gewirkt werden, dass jede Person auf sämtliche Therapiedaten eines Beatmungsgerätes 1 zugreifen kann, auch wenn sie nur für einen Teil der Daten eine Autorisierung oder die nötigen Kenntnisse besitzt.

### Bezugszeichenliste:

- 1: Beatmungsgerät
- 2: Datenverarbeitungseinrichtung
- 3: Gegenstelle
- 10: Beatmungssystem
- 11: Anzeigeeinrichtung
- 12: Code-Generator
- 13: Server
- 21: Überwachungseinrichtung
- 23: Personal Computer
- 31: Speichereinrichtung
- 41: Speichermedium
- 51: Übertragungseinrichtung
- 61: Benutzerschnittstelle
- 100: Beatmungseinrichtung
- 101: Gebläseeinrichtung
- 102: Atemschnittstelle
- 103: Bedienelemente
- 105: Atemmaske
- 106: Kopfhaube
- 107: Kupplungselement
- 108: Ausatmungselement
- 109: Verbindungsschlauch
- 110: Druckmessschlauch
- 111: Eingangsstutzen
- 112: Koppeleinrichtung

## Patentansprüche

1. Verfahren zum Betreiben wenigstens einer Datenverarbeitungseinrichtung (2) eines Beatmungssystems (10) mit wenigstens einem Beatmungsgerät (1) und mit wenigstens einer räumlich von dem Beatmungsgerät (1) getrennten Gegenstelle (3),
wobei mit der Datenverarbeitungseinrichtung (2) Therapiedaten zwischen dem Beatmungsgerät (1) und der Gegenstelle (3) wenigstens teilweise übertragbar sind und wobei von wenigstens einem Benutzer des Beatmungssystems (10) wenigstens ein Berechtigungscode in der Datenverarbeitungseinrichtung (2) hinterlegt wird, um über die Gegenstelle (3) einen berechtigten Zugriff auf die Therapiedaten zu erhalten,
und wobei der Berechtigungscode dem Benutzer erst dann zur Verfügung gestellt wird, wenn dieser den Berechtigungscode durch wenigstens eine gegenüber der Datenverarbeitungseinrichtung (2) vorzunehmende Benutzeraktion anfordert.

2. Verfahren nach dem vorhergehenden Anspruch, wobei die Benutzeraktion wenigstens teilweise mit dem Beatmungsgerät (1) durchgeführt wird und wobei der Berechtigungscode nur zu einem Zugriff auf die Therapiedaten desjenigen Beatmungsgerätes (1) berechtigt, mit dem die Benutzeraktion durchgeführt wird.

3. Verfahren nach einem der vorhergehenden Ansprüche, wobei die Benutzeraktion wenigstens eine Inbetriebnahme des Beatmungsgeräts (1) und vorzugsweise auch wenigstens eine Eingabe in das Beatmungsgerät (1) umfasst.

4. Verfahren nach einem der vorhergehenden Ansprüche, wobei die Benutzeraktion wenigstens eine biometrische Erfassung des Benutzers und/oder wenigstens ein Sprachkommando und/oder wenigstens eine Kopplung des Beatmungsgeräts (1) mit wenigstens einem Speichermedium (41) umfasst.

5. Verfahren nach einem der vorhergehenden Ansprüche, wobei dem Benutzer der angeforderte Berechtigungscode auf wenigstens einer Anzeigeeinrichtung (11) angezeigt wird und/oder auf wenigstens einem Speichermedium (41) abgelegt wird.

6. Verfahren nach einem der vorhergehenden Ansprüche, wobei die Benutzeraktion wenigstens eine Registrierung des Benutzers gegenüber der Datenverarbeitungseinrichtung (2), bevorzugt gegenüber der Gegenstelle (3) umfasst, sodass nur ein registrierter Benutzer den Berechtigungscode anfordern kann.

7. Verfahren nach dem vorhergehenden Anspruch, wobei dem Benutzer bei der Registrierung wenigstens ein Zertifikat zugewiesen wird und wobei das Zertifikat dem Beatmungsgerät (1) vorgelegt werden muss, um den Berechtigungscode anfordern zu können.

8. Verfahren nach dem vorhergehenden Anspruch, wobei das Zertifikat auf einem transportablen Speichermedium (41) abgelegt wird und wobei der Berechtigungscode nur angefordert werden kann, wenn das Beatmungsgerät (1) mit dem Speichermedium (41) gekoppelt ist.

9. Verfahren nach dem vorhergehenden Anspruch, wobei von der Gegenstelle (3) zusätzlich zu dem Zertifikat auch wenigstens ein das Beatmungsgerät (1) identifizierendes Gerätezertifikat auf dem Speichermedium (41) abgelegt wird.

10. Verfahren nach einem der drei vorhergehenden Ansprüche, wobei der Berechtigungscode wenigstens teilweise durch das Beatmungsgerät (1) unter Einbeziehung des Zertifikats generiert wird.

11. Verfahren nach Anspruch 6, wobei der Zugriff auf die Therapiedaten freigegeben wird, wenn sich der registrierte Benutzer bei der Gegenstelle (3) anmeldet, nachdem er zuvor als ein registrierter Benutzer den Berechtigungscode angefordert hat oder wenn der Benutzer den ihm zur Verfügung gestellten Berechtigungscode der Gegenstelle (3) mitteilt.

12. Verfahren nach dem vorhergehenden Anspruch, wobei der Berechtigungscode durch den Benutzer in der Gegenstelle (3) eingegeben wird oder das Beatmungsgerät (1) den Berechtigungscode wenigstens teilweise selbstständig an die Gegenstelle (3) übermittelt.

13. Verfahren nach einem der vorhergehenden Ansprüche, wobei der Berechtigungscode wenigstens eine Seriennummer und/oder wenigstens eine Geräteidentifikationsnummer des Beatmungsgeräts (1) umfasst oder wenigstens teilweise aus daraus generiert wird.

14. Verfahren nach einem der vorhergehenden Ansprüche, wobei der Berechtigungscode wenigstens eine Information über eine Autorisierung des Benutzers hinsichtlich des Zugriffs auf die Therapiedaten und/oder wenigstens eine Information über wenigstens ein Leserecht und/oder Schreibrecht des Benutzers umfasst.

15. Verfahren nach einem der vorhergehenden Ansprüche, wobei der Berechtigungscode wenigstens eine zeitlich begrenzte Gültigkeit und/oder eine einmalige Gültigkeit aufweist.

16. Verfahren nach dem vorhergehenden Anspruch, wobei die Gültigkeit des Berechtigungscodes an den Benutzer gekoppelt ist oder die Gültigkeit des Berechtigungscodes aufgehoben wird, wenn das Beatmungsgerät (1) für einen neuen Patienten eingesetzt wird.

17. Verfahren nach einem der vorhergehenden Ansprüche, wobei der Berechtigungscode, welcher vom Benutzer angefordert wird, wenigstens teilweise in dem Beatmungsgerät (1) hinterlegt ist und/oder wenigstens teilweise durch das Beatmungsgerät (1) generiert wird.

18. Verfahren nach einem der vorhergehenden Ansprüche, wobei wenigstens ein Teil des Berechtigungscodes, welcher vom Benutzer angefordert wird, räumlich getrennt von dem Beatmungsgerät (1), insbesondere in der Gegenstelle (3), generiert wird und/oder hinterlegt ist.

19. Beatmungssystem (10), umfassend wenigstens ein Beatmungsgerät (1) mit wenigstens einer Beatmungseinrichtung (100) zur Erzeugung eines Luftstroms für eine Atemtherapie und wenigstens eine räumlich von dem Beatmungsgerät (1) getrennte Gegenstelle (3) und wenigstens eine Datenverarbeitungseinrichtung (2),
wobei mit der Datenverarbeitungseinrichtung (2) Therapiedaten zwischen dem Beatmungsgerät (1) und der Gegenstelle (3) übertragbar sind
und wobei in der Datenverarbeitungseinrichtung (2) wenigstens ein Berechtigungscode hinterlegbar ist, um einem Benutzer über die Gegenstelle (3) einen berechtigten Zugriff auf die Therapiedaten zu gewähren,
und wobei die Datenverarbeitungseinrichtung (2) dazu geeignet und ausgebildet ist, dem Benutzer den Berechtigungscode zur Verfügung zu stellen, wenn dieser den Berechtigungscode durch wenigstens eine gegenüber der Datenverarbeitungseinrichtung (2) vorzunehmende Benutzeraktion anfordert.

## Claims

1. A method for operating at least one data processing apparatus (2) of a ventilation system (10) having at least one ventilator (1) and having at least one remote station (3) spatially separated from the ventilator (1),
wherein therapy data can be at least partly transmitted between the ventilator (1) and the remote station (3) with the data processing apparatus (2),
and wherein at least one authorization code is recorded by at least one user of the ventilation system (10) in the data processing apparatus (2) in order to obtain authorized access to the therapy data via the remote station (3), and wherein the authorization code is only provided to the user when the latter requests the authorization code by at least one user action to be undertaken with respect to the data processing apparatus (2).

2. The method according to the preceding claim, wherein the user action is performed at least partly with the ventilator (1) and wherein the authorization code only authorizes access to the therapy data of that ventilator (1) with which the user action is performed.

3. The method according to any one of the preceding claims, wherein the user action comprises at least one commissioning of the ventilator (1) and preferably also at least one entry in the ventilator (1).

4. The method according to any one of the preceding claims, wherein the user action comprises at least one biometrical detection of the user and/or at least one voice command and/or at least one connecting of the ventilator (1) to at least one storage medium (41).

5. The method according to any one of the preceding claims, wherein the requested authorization code is indicated to the user on at least one display apparatus (11) and/or stored on at least one storage medium (41).

6. The method according to any one of the preceding claims, wherein the user action comprises at least one registration of the user with respect to the data processing apparatus (2), preferably with respect to the remote station (3), so that only a registered user can request the authorization code.

7. The method according to the preceding claim, wherein the user is assigned at least one certificate upon registration and wherein the certificate must be presented to the ventilator (1) in order to be able to request the authorization code.

8. The method according to the preceding claim, wherein the certificate is stored on a portable storage medium (41) and wherein the authorization code may only be requested when the ventilator (1) is connected to the storage medium (41).

9. The method according to the preceding claim, wherein in addition to the certificate, the remote station (3) also stores at least one equipment certificate identifying the ventilator (1) on the storage medium (41).

10. The method according to any one of the three preceding claims, wherein the authorization code is generated at least partly by the ventilator (1), taking into account the certificate.

11. The method according to Claim 6, wherein access to the therapy data is released when the registered user logs in to the remote station (3) after having first requested the authorization code as a registered user or when the user communicates the authorization code provided to him to the remote station (3).

12. The method according to the preceding claim, wherein the authorization code is entered by the user in the remote station (3) or the ventilator (1) relays the authorization code at least partly independently to the remote station (3).

13. The method according to any one of the preceding claims, wherein the authorization code comprises at least one serial number and/or at least one equipment identification number of the ventilator (1) or is generated at least partly from the latter.

14. The method according to any one of the preceding claims, wherein the authorization code comprises at least one piece of information about an authorization of the user with regard to access to the therapy data and/or at least one piece of information about at least one read right and/or write right of the user.

15. The method according to any one of the preceding claims, wherein the authorization code has at least a time-limited validity and/or a one-time validity.

16. The method according to the preceding claim, wherein the validity of the authorization code is connected to the user or the validity of the authorization code is cancelled when the ventilator (1) is deployed for a new patient.

17. The method according to any one of the preceding claims, wherein the authorization code which is requested by the user is at least partly recorded in the ventilator (1) and/or is at least partly generated by the ventilator (1).

18. The method according to any one of the preceding claims, wherein at least a part of the authorization code which is requested by the user is generated and/or recorded, spatially separated from the ventilator (1), in particular in the remote station (3).

19. A ventilation system (10), comprising at least one ventilator (1) having at least one ventilation apparatus (100) to create an air flow for a respiratory therapy and at least one remote station (3) spatially separated from the ventilator (1) and at least one data processing apparatus (2),
wherein therapy data can be transmitted between the ventilator (1) and the remote station (3) with the data processing apparatus (2),
and wherein at least one authorization code can be stored in the data processing apparatus (2) in order to grant a user authorized access to the therapy data via the remote station (3), and wherein the data processing apparatus (2) is suitable and designed for providing the user with the authorization code when the latter requests the authorization code by at least one user action to be undertaken with respect to the data processing apparatus (2).

## Revendications

1. Procédé d'exploitation d'au moins un dispositif de traitement de données (2) d'un système de ventilation (10) avec au moins un respirateur (1) et avec au moins un poste terminal (3) séparé spatialement du respirateur (1), dans lequel le dispositif de traitement de données (2) permet de transmettre au moins en partie des données thérapeutiques entre le respirateur (1) et le poste terminal (3) et dans lequel au moins un code d'autorisation est enregistré dans le dispositif de traitement de données (2) par au moins un utilisateur du système de ventilation (10) pour obtenir un accès autorisé aux données thérapeutiques via le poste terminal (3), et dans lequel le code d'autorisation n'est mis à la disposition de l'utilisateur qu'à partir du moment où ce dernier sollicite le code d'autorisation par au moins une action de l'utilisateur à accomplir auprès du dispositif de traitement de données (2).

2. Procédé selon la revendication précédente, dans lequel l'action de l'utilisateur est exécutée au moins en partie avec le respirateur (1) et dans lequel le code d'autorisation n'habilite qu'à accéder aux données thérapeutiques du respirateur (1), avec lequel l'action de l'utilisateur est exécutée.

3. Procédé selon l'une des revendications précédentes, dans lequel l'action de l'utilisateur comprend au moins une mise en service du respirateur (1) et de préférence aussi au moins une saisie dans le respirateur (1).

4. Procédé selon l'une des revendications précédentes, dans lequel l'action de l'utilisateur comprend au moins un enregistrement biométrique de l'utilisateur et/ou au moins une commande vocale et/ou au moins une connexion du respirateur (1) avec au moins un support de stockage (41).

5. Procédé selon l'une des revendications précédentes, dans lequel le code d'autorisation sollicité est indiqué à l'utilisateur sur au moins un afficheur (11) et/ou est enregistré sur au moins un support de stockage (41).

6. Procédé selon l'une des revendications précédentes, dans lequel l'action de l'utilisateur comprend au moins un enregistrement de l'utilisateur auprès du dispositif de traitement de données (2), de préférence auprès du poste terminal (3), de sorte que seul un utilisateur enregistré peut solliciter le code d'autorisation.

7. Procédé selon la revendication précédente, dans lequel au moins un certificat est attribué à l'utilisateur lors de l'enregistrement et dans lequel le certificat doit être soumis au respirateur (1) pour pouvoir solliciter le code d'autorisation.

8. Procédé selon la revendication précédente, dans lequel le certificat est enregistré sur un support de stockage transportable (41) et dans lequel le code d'autorisation ne peut être sollicité que si le respirateur (1) est connecté au support de stockage (41).

9. Procédé selon la revendication précédente, dans lequel au moins un certificat d'appareil identifiant le respirateur (1) est aussi enregistré sur le support de stockage (41) par le poste terminal (3) en plus du certificat.

10. Procédé selon l'une des trois revendications précédentes, dans lequel le code d'autorisation est généré au moins en partie par le respirateur (1) compte tenu du certificat.

11. Procédé selon la revendication 6, dans lequel l'accès aux données thérapeutiques est débloqué, si l'utilisateur enregistré se connecte au poste terminal (3), après qu'il a sollicité au préalable le code d'autorisation en tant qu'utilisateur enregistré ou si l'utilisateur communique le code d'autorisation mis à sa disposition au poste terminal (3).

12. Procédé selon la revendication précédente, dans lequel le code d'autorisation est saisi par l'utilisateur dans le poste terminal (3) ou le respirateur (1) transmet le code d'autorisation au moins en partie de façon autonome au poste terminal (3).

13. Procédé selon l'une des revendications précédentes, dans lequel le code d'autorisation comprend au moins un numéro de série et/ou au moins un numéro d'identification du respirateur (1) ou est généré au moins en partie à partir de ceux-ci.

14. Procédé selon l'une des revendications précédentes, dans lequel le code d'autorisation comprend au moins une information relative à une autorisation de l'utilisateur quant à l'accès aux données thérapeutiques et/ou au moins une information relative au moins à un droit de lecture et/ou à un droit d'écriture de l'utilisateur.

15. Procédé selon l'une des revendications précédentes, dans lequel le code d'autorisation présente au moins une validité limitée dans le temps et/ou une validité unique.

16. Procédé selon la revendication précédente, dans lequel la validité du code d'autorisation est liée à l'utilisateur ou la validité du code d'autorisation est annulée, si le respirateur (1) est employé pour un nouveau patient.

17. Procédé selon l'une des revendications précédentes, dans lequel le code d'autorisation, lequel est sollicité par l'utilisateur, est enregistré au moins en partie dans le respirateur (1) et/ou est généré au moins en partie par le respirateur (1).

18. Procédé selon l'une des revendications précédentes, dans lequel au moins une partie du code d'autorisation, lequel est sollicité par l'utilisateur, est générée et/ou enregistrée dans l'espace séparément du respirateur (1), en particulier dans le poste terminal (3).

19. Système de ventilation (10) comprenant au moins un respirateur (1) avec au moins un dispositif de ventilation (100) pour générer un flux d'air destiné à une thérapie respiratoire et au moins un poste terminal (3) séparé spatialement du respirateur (1) et au moins un dispositif de traitement de données (2), dans lequel le dispositif de traitement de données (2) permet de transmettre des données thérapeutiques entre le respirateur (1) et le poste terminal (3) et dans lequel au moins un code d'autorisation peut être enregistré dans le dispositif de traitement de données (2) pour accorder un accès autorisé aux données thérapeutiques à un utilisateur via le poste terminal (3), et dans lequel le dispositif de traitement de données (2) est apte et conçu de manière à mettre le code d'autorisation à la disposition de l'utilisateur lorsque ce dernier sollicite le code d'autorisation par une action de l'utilisateur à accomplir auprès du dispositif de traitement de données (2).
